Europäisches Patentamt

European Patent Office

Office européen des brevets

(19)

(11) Veröffentlichungsnummer : **0 372 452 B1**

# (12) EUROPÄISCHE PATENTSCHRIFT

(45) Veröffentlichungstag der Patentschrift :
**10.06.92 Patentblatt 92/24**

(21) Anmeldenummer : **89122318.2**

(22) Anmeldetag : **04.12.89**

(30) Priorität : **02.12.88 DE 3840714**

(43) Veröffentlichungstag der Anmeldung :
**13.06.90 Patentblatt 90/24**

(45) Bekanntmachung des Hinweises auf die
Patenterteilung :
**10.06.92 Patentblatt 92/24**

(84) Benannte Vertragsstaaten :
**CH FR GB IT LI NL SE**

(56) Entgegenhaltungen :
**EP-A- 0 154 680**
**US-A- 3 073 305**
**US-A- 3 674 023**
**US-A- 4 727 863**

(51) Int. Cl.⁵ : **A61F 5/04**

(54) **Sprunggelenkorthese.**

(73) Patentinhaber : **Bauerfeind GmbH & Co.**
**Arnoldstrasse 15**
**W-4152 Kempen 1 (DE)**

(72) Erfinder : **Hess, Heinrich, Prof.Dr.med.**
**Kapuzinerstrasse 4**
**W-6630 Saarlouis (DE)**
Erfinder : **Krause, Wolfgang, Prof.Dr.med.**
**Wilhelmhöher Allee 345**
**W-3500 Kassel (DE)**
Erfinder : **Bauerfeind, Hans B.**
**Wiesenstrasse 18**
**W-4152 Kempen 1 (DE)**

(74) Vertreter : **Bardehle, Heinz, Dipl.-Ing. et al**
**Patent- und Rechtsanwälte**
**Bardehle-Pagenberg-Dost-Altenburg-Frohwitter-**
**Geissler & Partner Postfach 86 06 20**
**W-8000 München 86 (DE)**

EP 0 372 452 B1

EP 0 372 452 B1

## Beschreibung

Die Erfindung bezieht sich auf eine Sprunggelenkorthese, die einen U-förmigen Stützbügel enthält, dessen Schenkel unterhalb des Fußes in einem Steg zusammenlaufen, über die Knöchel hinausreichen und in ihrem Endbereich durch ein Befestigungsband zusammengehalten sind.

Eine derartige Orthese ist aus der DE-OS 34 35 955 bekannt. Sie besteht im wesentlichen aus einem U-förmigen Stützbügel, dessen Schenkel über die Knöchel nach oben verlaufen und in ihren Endbereichen durch ein Klettband umfaßt sind, das die Enden der Schenkel gegen das Bein druckt. Die Schenkel laufen unterhalb des Fußes in einem Steg zusammen, der sich vom Mittelfuß bis in den Bereich der Ferse erstreckt.

Es hat sich in der Praxis gezeigt, daß mit derartigen Orthesen der Fuß in seiner Rechtwinkel-Stellung zwar gegen rein seitliches Umknicken etwa um eine in Fußrichtung liegende Achse geschützt werden kann, wodurch jedoch die häufigen Fälle des Gleit-Knickens seitlich und gleichzeitig nach vorn nicht erfaßt werden. Abgesehen davon erfordert die bekannte Orthese, deren stabilisierende Wirkung allein von dem U-förmigen Stützbügel ausgeht, eine erhebliche Biegesteifigkeit, was nur durch relativ starke Schenkel erzielt werden kann.

Der Erfindung liegt die Aufgabe zugrunde, eine Sprunggelenkorthese zu schaffen, die das Umknicken vor allem in Richtung seitlich-vorn, also in Richtung auf eine Spitzfuß-Stellung, verhindert. Erfindungsgemäß geschieht dies dadurch, daß der äußere Schenkel seitlich vor seinem Knöchel und der innere Schenkel in Gegenüberstellung zum äußeren Schenkel vor der Achillessehne hochgeführt ist, die Schenkel in Richtung zum Steg bis zu einer Lage vor der Ferse geführt sind und in Richtung zu ihren Enden aufwärts derart verlaufen, daß sie seitlich neben den Schienbeinkanten etwa parallel zu dießen aufwärts streben, und daß im unteren Bereich der Schenkel ein Halteband, insbesondere Klettband, angebracht ist, das von dem einen Schenkel über den Rist schräg aufwarts zum anderen Schenkel an diesem festlegbar verläuft, oberhalb der Knöchel um die Achillessehne greift und auf dem Rist sich überkreuzend an dem anderen Schenkel in einem Halteteil endet.

Der äußere Schenkel folgt im Bereich unterhalb des Knöchels etwa der Richtung des vorderen Außenbandes, das erfahrungsgemäß beim Umknicken seitlich nach vorn einer besonderen Beanspruchung ausgesetzt wird und durch diese funktionell-anatomische Schenkelführung besonders geschützt wird. Durch die Gegenüberstellung beider Schenkel ergibt sich eine sichere Erfassung deß Sprunggelenks. Die Schenkel des Stützbügels sind damit an die Anatomie der besonders gefährdeten Bänder günstig angepaßt. Aufgrund des Verlaufes der Schenkel in Richtung zum Steg bis zu einer Lage vor der Ferse und in Richtung zu ihren Enden neben den Schienbeinkanten ergibt sich eine Fluchtlinie über die Enden zum Steg, die im Effekt der Richtung des Beines über die Knöchel zur Fußsohle folgt, so daß durch diese Formgebung auch dem rein seitlichen Umknicken des Fußes entgegengewirkt wird. Weiterhin liefert das das Sprunggelenk im Bereich des Ristes und der Achillessehne umfassende Halteband eine zusätzliche Stützfunktion, da es aufgrund der Kreuzführung über dem Rist dem eigentlichen funktionell-pathologischen Gleit-Knicken nach vorn-seitlich entgegenwirkt. Es liegt also hinsichtlich der Stützung des Sprunggelenks eine Kombinationswirkung zwischen dem kreuzförmig geführten Halteband und der besonderen Gestaltung des U-förmigen Stützbügels vor.

Die Anordnung des Haltebandes gestaltet man zweckmäßig so, daß dieses mit seinem einen Ende an dem einen Schenkel durch Klebung, Nietung oder dergleichen befestigt ist, mit seinem anderen Ende durch einen am anderen Schenkel befestigten Regulierring schlaufenförmig gezogen und durch Umlegen an einer Klettfläche des Haltebandes gesichert ist, wobei die Festlegung am anderen Schenkel mittels eines Klettverschlusses erfolgt. Beim Anlegen des Haltebandes ergibt sich damit praktisch automatisch eine Fixierung der beiden Schenkel gegeneinander unter Aufnahme von Zugspannungen, die sowohl in Richtung der Schenkel als auch quer zu diesen verlaufen, da im Ristbereich das Halteband durch seine Kreuzführung in die Schenkel einläuft und den jeweils anderen Schenkel überkreuzt. Beim Anlegen des Haltebandes wird dieses an dem betreffenden anderen Schenkel festgelegt, wozu insbesondere eine an dem Schenkel angebrachte Klettfläche dient.

Es hat sich sowohl für das Anlegen der Sprunggelenkorthese als auch für ihre medizinische Wirkung als günstig erwiesen, die Befestigungsstelle des einen Endes des Haltebandes am äußeren Sc-henkel, die des jeweils anderen Endes am inneren Schenkel und den Regulierring ebenfalls an den inneren Schenkel anzubringen.

Das Befestigungsband läßt sich zweckmäßig dazu benutzen, die beiden Enden der Schenkel in einem gewünschten Abstand zu halten. Hierzu wird am Ende des einen Schenkels ein Klettband als Befestigungsband angebracht, das in Richtung zum anderen Schenkel verläuft und diesen überquerend mittels einer Klettfläche in gewünschtem Abstand zu ersterem Schenkel hält. Beim Anlegen des als Befestigungsband wirkenden Klettbandes, bei dem das Klettband um das Bein geschlungen wird, überquert dieses, ausgehend von seiner Befestigungsstelle am Ende des einen Schenkels, den anderen Schenkel, wobei es sich mit der an dieser Stelle angebrachten Klettfläche verhakt, wodurch sich ein durch das Anlegen des Klettbandes definierter Abstand zwischen den beiden Enden der Schenkel ergibt.

2

Zweckmäßig gestaltet man den äußeren Schenkel so, daß dieser als teilweise Umrundung um den Außenknöchel herum verläuft. Herdurch ergibt sich ein Verlauf des Schenkels über den Rist des Fußes schräg nach innen aufwärts, wodurch zusätzlich eine Abstützung der vorderen Außenbänder erzielt wird und damit auf diese treffende Überlastungen aufgenommen werden.

In den Figuren ist ein Ausführungsbeispiel der Erfindung dargestellt. Es zeigen

Figure 1: eine Ansicht der Srpunggelenkorthese von der Zehenseite eines rechten Fusses her gesehen,

Figur 2: die gleiche Orthese am Fuß von der Außenseite her gesehen,

Figur 3: die gleiche Orthese am Fuß von der Innenseite her gesehen,

Figur 4: die gleiche Orthese, am Fuß, in perspektivischer Darstellung von der Zehenseite her gesehen.

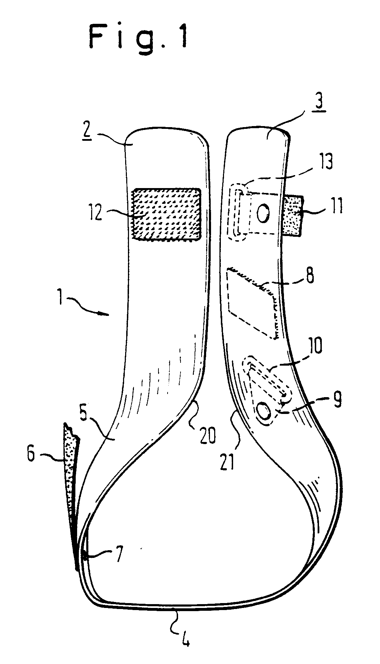

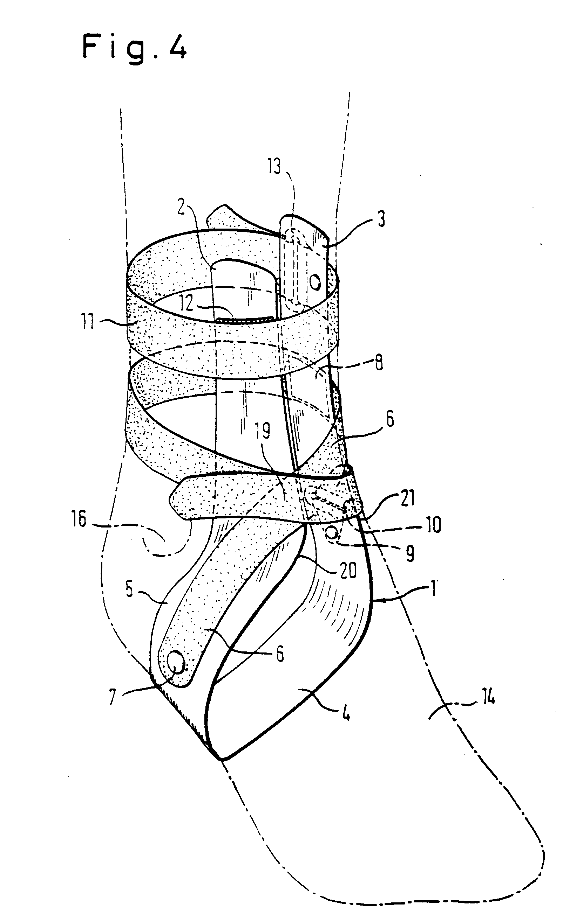

Die in Figur 1 dargestellte Sprunggelenkorthese enthält den U-förmigen Stützbügel 1, der aus den beiden Schenkeln 2 und 3 sowie dem die Schenkel verbindenden Steg 4 besteht. Im unteren Bereich 5 des Schenkels 2 ist das aus dem Klettband 6 bestehende Halteband angebracht, das hier durch den Niet 7 mit dem Schenkel 2 fest verbunden ist. Aus Gründen der Übersichtlichkeit der Darstellung ist das Klettband abgebrochen gezeichnet. Im Bereich oberhalb des Ristes ist am Schenkel 3 die Klettfläche 8 vorgesehen, die aus einem aufgeklebten Stück Klettverschluß besteht. Über diese Klettfläche 8 wird beim Anlegen der Orthese das Klettband 6 gelegt, was anhand der Figur 4 näher erläutert wird. Unterhalb der Klettfläche 8 ist die zweite Befestigungsstelle für das angelegte Klettband 6 in Form der angenieteten Lasche 9 mit daran befestigtem Regulierring 10 vorgesehen. Beim Anlegen des Klettbandes 6 wird dieses durch den Regulierring gezogen und mittels Klettverschluß gehalten, worauf ebenfalls im Zusammenhang mit der Figur 4 näher eingegangen wird.

Am oberen Ende des Schenkels 3 ist das eine Ende des Befestigungsbandes 11 angebracht, das hier aus Gründen der Übersichtlichkeit der Darstellung abgebrochen gezeichnet ist. Es verläuft in Richtung auf den anderen Schenkel 2, an dessen Ende gegenüberliegend der Befestigungsstelle des Befestigungsbandes 11 die Klettfläche 12 angebracht ist, z.B. durch Aufkleben eines Klettverschlusses. Beim Anlegen des Befestigungsbandes 11 wird dieses über die Klettfläche 12 gelegt und hält sich an dieser fest. Hierauf wird weiter unten im Zusammenhang mit der Figur 4 näher eingegangen. Das Befestigungsband 11 endet an seiner rückwärtigen Seite in dem Regulierring 13, in den das betreffende andere Ende des Befestigungsbandes 11 nach seinem Anlegen eingeführt und um diesen herumgeschlungen wird.

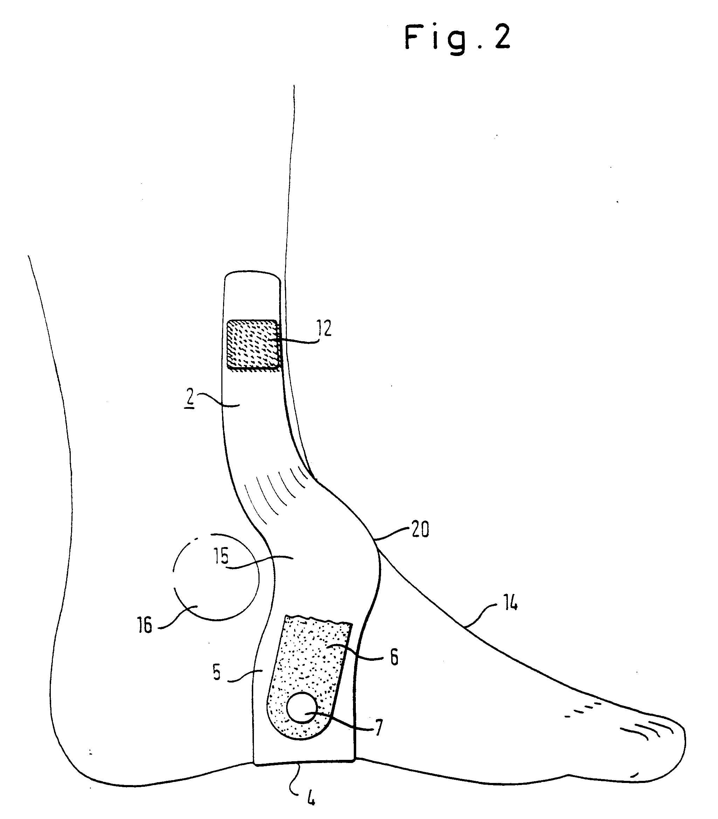

Die in der Figur 1 dargestellte Sprunggelenkorthese ist in Fig. 2 von der Seite gesehen an einem rechten Fuß 14 angelegt dargestellt, und zwar von der Außenseite des Fußes 14 her gesehen. Wie ersichtlich, läuft der Schenkel 2 als teilweise Umrundung 15 um den als Kreis symbolisch angedeuteten Außenknöchel 16 herum.

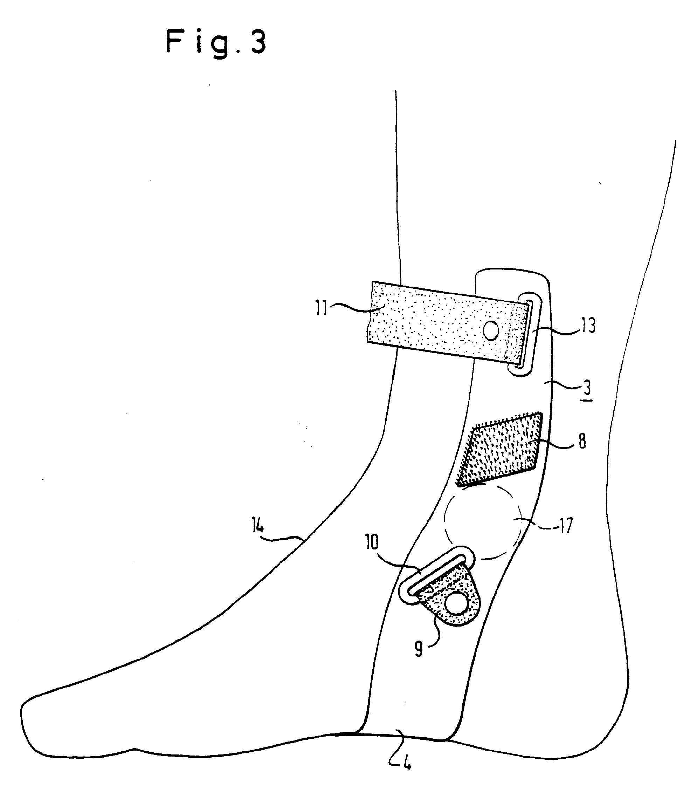

In Figur 3 ist die gleiche Orthese am rechten Fuß 14 angelegt wiedergegeben, und zwar von der Innenseite des Fußes 14 her gesehen. Der hier sichtbare Schenkel 3 verläuft vor der Achillessehne über den Innenknöchel 17. An ihm ist mittels der angenieteten Lasche 9 der Regulierring 10 angebracht, der zur Befestigung des betreffenden Endes des Klettbandes 6 (siehe Figur 1) dient. Oberhalb des Regulierringes 10 ist die Klettfläche 8 angebracht, auf deren Funktion im Zusammenhang mit den Figuren 1 und 2 bereits eingegangen worden war. Im Bereich des oberen Endes des Schenkels 3 ist das eine Ende des Befestigungsbandes 11 angenietet, das hier aus Gründen der Übersichtlichkeit der Darstellung abgebrochen gezeichnet ist. Das Befestigungsband 11 endet nach rückwärts in dem Regulierring 13, in den das Befestigungsband 11 nach Umschlingen des Beines eingezogen wird.

Bezüglich der Figuren 2 und 3 sei noch darauf hingewiesen, daß diese auch den Steg 4 zeigen, der hier unmittelbar vor dem Bereich der Ferse des Fußes 14 liegt.

In der Figur 4 ist in teilweiser Wiederholung der Darstellung gemäß Figur 1 die an einem rechten Fuß 14 angelegte Sprunggelenkorthese 1 wiedergegeben.

Das Klettband 6 wird ausgehend von der Befestigungsstelle mit dem Niet 7 nach vorn über den Rist des Fußes 14 geführt und gelangt dabei, den Schenkel 3 im Bereich oberhalb des Ristes kreuzend, auf die Klettfläche 8, von der das Klettband 6 gehalten wird. Das Klettband 6 ist sodann um den Fuß 14 nach hinten herum geschlungen, und zwar oberhalb der Knöchel 16 (17 nicht sichtbar) um die Achillessehne. Das Klettband 6 wird dann auf dem Rist über die Kreuzungsstelle 19 zum Regulierring 10 geführt, durch den es hindurchgeschlungen ist und durch rückwärtiges Umlegen und Andrücken gegen den darunterliegenden Teil des Klettbandes 6 gehalten wird. Hierzu dient ebenfalls ein an dieser Stelle vorgesehener Klettverschluß.

Es ergibt sich damit eine Stützung des Sprunggelenkes in mehrerlei Hinsicht. Einerseits verlaufen die Schenkel 2 und. 3, wie aus den Figuren 2 und 3 ersichtlich, in Gegenüberstellung in Längsrichtung des Beines seitlich neben den Schienbeinkanten. Im Effekt liegt eine durchgehende, im wesentlichen in Längsrichtung des Beines verlaufende Abstützung durch die Schenkel 2 und 3 vor, so daß ein rein seitliches Umknicken des Fußes, wie oben dargelegt, praktisch vollständig aufgefangen wird. Darüberhinaus erbringt die teilweise Umrundung 15 die wesentliche Wirkung, daß der Übergangsbereich 20 über den Rist schräg nach innen aufwärts verläuft, womit der äußere Bereich 20 etwa in Richtung der vorderen Außenbänder verläuft und damit auf diese treffende Überlastungen aufzunehmen vermag. Dabei wird diese Aufnahmefähigkeit für derartige Spannungen durch das Klettband 6 entscheidend unterstutzt, das nämlich einerseits der Richtung dieses Bereichs folgt und nach Umschlingung des Fußes sich selbst kreuzend in den gegenüberliegenden Bereich einläuft und dort auftretende Zugspannungen auffängt. Schließlich bewirkt die sich kreuzende Umschlingung des Fußes durch das Klettband 6 eine feste Verbindung zwischen Fuß 14 und den Schenkeln 2 und 3.

Das an den Enden der Schenkel 2 und 3 angebrachte Befestigungsband wird, wie Figur 4 deutlich zeigt, dazu ausgenutzt, die Enden der Schenkel 2 und 3 im gewünschten Abstand zu halten, was letztlich darauf hinausläuft, daß die beiden Schenkel 2 und 3 etwa parallel zueinander seitlich der Schienbeinkanten verlaufen und in dieser Lage gehalten werden. Das zweckmäßigerweise ebenfalls als Klettband ausgebildete Befestigungsband 11, das, in Richtung auf den Schenkel 2 weisend, am Schenkel 3 befestigt ist, wird über den Schenkel 2 gelegt und drückt dabei gegen die Klettfläche 12, von der dann das Befestigungsband 11 gehalten wird. Hierdurch ergibt sich ein definierter Abstand zwischen den beiden Endes der Schenkel 2 und 3. Das Befestigungsband 11 wird sodann um das Bein herumgeschlungen, in den Regulierring 13 eingezogen und nach hinten umgeknickt, wobei es gegen den darunterliegenden Bereich des Befestigungsbandes 11 gedrückt wird und dabei mittels Klettverschluß an diesem haftet.

## Patentansprüche

1. Sprunggelenkorthese, die einen aus biegsamem Material bestehenden U-förmigen Stützbügel (1) enthält, dessen Schenkel (2, 3) unterhalb des Fußes (14) in einem Steg (4) zusammenlaufen, über die Knöchel (16, 17) hinausreichen und in ihrem Endbereich durch ein das Bein umschlingendes ßefestigungsband (11) zusammengehalten sind, **dadurch gekennzeichnet**, daß der äußere Schenkel (2) seitlich vor seinem Knöchel (16) und der innere Schenkel (3) in Gegenüberstellung zum äußeren Schenkel (2) vor der Achillessehne hochgeführt ist, die Schenkel (2, 3) in Richtung zum Steg (4) bis zu einer Lage vor der Ferse geführt sind und in Richtung zu ihren Enden aufwärts derart verlaufen, daß sie seitlich neben den Schienbeinkanten etwa parallel zu diesen aufwärts streben, und daß im unteren Bereich der Schenkel (2, 3) ein Halteband (6), insbesondere Klettband, angebracht ist, das von dem einen Schenkel (2) über den Rist schräg aufwärts zum anderen Schenkel (3) an diesem festlegbar verläuft, oberhalb der Knöchel (16, 17) um die Achillessehne greift und auf dem Rist sich überkreuzend an dem anderen Schenkel (3) in einem Halteteil (10) endet.

2. Sprunggelenkorthese nach Anspruch 1, dadurch gekennzeichnet, daß das Halteband (6) mit seinem einen Ende an dem einen Schenkel (2) durch Klebung, Nietung (7) oder dergleichen befestigt ist, mit seinem anderen Ende durch einen am anderen Schenkel (3) befestigten Regulierring (10) schlaufenförmig gezogen und durch Umlegen an einer Klettfläche des Haltebandes (6) gesichert ist, wobei die Festlegung am anderen Schenkel (3) mittels eines Klettverschlusses (8) erfolgt.

3. Sprunggelenkorthese nach Anspruch 1 oder 2, dadurch gekennzeichnet, daß die Befestigungsstelle (7) des einen Endes des Haltzbandes (6) am äußeren Schenkel (2) angebracht ist und das andere Ende des Haltebandes (6) am inneren Schenkel (3) festgelegt (8) ist und der Regulierring (10) ebenfalls am inneren Schenkel (3) angebracht ist.

4. Sprunggelenkorthese nach einem der Ansprüche 1 bis 3, dadurch gekennzeichnet, daß am ende des einen Schenkels (3) ein Klettband als Befestingunsband (11) angebracht ist, das in Richtung zum anderen Schenkel (2) verläuft und diesen überquerend mittels einer Klettfläche (12) in gewünschtem abstand zu ersterem Schenkel (3) hält.

5. Sprunggelenkorthese nach einem der Ansprüche 1 bis 4, **dadurch gekennzeichnet,** daß der äußere Schenkel (2) als teilweise Umrundung (15) um den Außenknöchel (16) herum verläuft.

## Claims

1. Ankle support comprising a U-shaped support bandage (1) consisting of flexible material, the side portions (2, 3) of which are connected to one another by means of a bridge (4) under the foot (14), extend beyond the ankle bones (16, 17) and are held together in their end region by a fixing strip (11) embracing the leg, characterised in that the outer side portion (2) is passed laterally up in front of its ankle bone (16) and the inner side portion (2) is passed up in front of the Achilles tendon opposite the outer side portion (2), the side portions (2, 3) are guided in the direction of the bridge (4) to a position in front of the heel and extend upwards in the direction of their ends in such a manner that they are urged upwards laterally alongside the shins substantially parallel thereto, and that a retaining strip (6), especially a Velcro strip is applied in the lower region of the side portions (2, 3), wherein said strip extends diagonally upwards via the instep from one side portion (2) to the other side portion (3) and can be fixed thereto, embraces the Achilles tendon above the ankle bones (16, 17) and, crossing over the instep, ends in a retaining portion (10) on the other side portion (3).

2. Ankle support according to claim 1, characterised in that the retaining strip (6) is fixed by one of its ends to one side portion (2) by gluing, riveting (7) or the like and by its other end is looped through an adjusting ring (10) fixed to the other side portion (3) and is placed securely on a Velcro surface of the retaining strip (6), fixing at the other side portion (3) being effected by means of a Velcro strip fastener (8).

3. Ankle support according to claim 1 or claim 2, characterised in that the fixing point (7) of one end of the retaining strip (6) is applied to the outer side portion (2) and the other end of the retaining strip (6) is fixed (8) to the inner side portion (3) and the adjusting ring (10) is also applied to the inner side portion (3).

4. Ankle support according to one of claims 1 to 3, characterised in that a Velcro strip is applied as a fixing strip (11) to the end of one side portion (3), said strip extending in the direction of the other side portion (2) and, traversing the latter, keeping it at the desired distance from the first side portion (3) by means of a Velcro surface (12).

5. Ankle support according to one of claims 1 to 4, characterised in that the outer side portion (2) extends around the external ankle bone (16) partially surrounding (15) it.


## Revendications

1. Chevillière, comprenant un étrier de soutien (1) en U, réalisé en matériau flexible, dont les branches (2, 3) rejoignent une partie centrale (4) en dessous du pied (14), dépassent les malléoles (16, 17) et sont maintenues ensemble, dans leur région terminale, par une bande de fixation (11) entourant la jambe, **caractérisée** en ce que la branche extérieure (2) monte latéralement devant la malléole externe (16) et la branche intérieure (3) monte devant le tendon d'Achille en vis-à-vis de la branche extérieure (2), les branches (2, 3) étant amenées, en direction de la partie centrale (4), jusqu'à une position située en avant du talon et s'étendant vers le haut, en direction de leurs extrémités, de telle sorte qu'elles s'élancent latéralement à côté des arêtes du tibia et environ parallèlement à ces dernières, et en ce qu'une bande de maintien (6), notamment une bande agrippante, est fixée dans la région inférieure des branches (2, 3) et monte en oblique, sur le cou de pied, de la première branche (2) vers l'autre branche (3) en pouvant être fixée en position sur cette dernière, puis entoure le tendon d'Achille au-dessus des malléoles (16, 17) pour atteindre enfin, sur le coup de pied et en se croisant elle-même, une pièce de fixation (10) prévue sur l'autre branche (3).

2. Chevillière selon la revendication 1, caractérisée en ce que la bande de maintien (6) est, par sa première extrémité, fixée sur la première branche (2) par collage, rivetage (7) ou similaire et, par son autre extrémité, passée en boucle dans un anneau de réglage (10) fixé sur l'autre branche (3), en étant assujettie par rabattement sur une surface agrippante de la bande de maintien (6), la fixation en position sur cette autre branche (3) s'effectuant au moyen d'un ruban agrippant (8).

3. Chevillière selon la revendication 1 ou 2, caractérisée en ce le point de fixation (7) de la première extrémité de la bande de maintien (6) est prévu sur la branche extérieure (2), et l'autre extrémité de la bande de maintien (6) est fixée en position 18) sur la branche intérieure (3), l'anneau de réglage (10) étant également prévu sur la branche intérieure (3).

4. Chevillière selon l'une des revendications 1 à 3, caractérisée en ce qu'une bande agrippante est fixée à l'extrémité de ladite autre branche (3), comme bande de fixation (11) qui s'étend en direction de la première branche (2) et, en croisant cette dernière sur une surface agrippante (12), la maintient à la distance souhaitée de ladite autre branche (3).

5. Chevillière selon l'une des revendications 1 à 4, caractérisée en ce que la branche extérieure (2) contourne la malléole externe (16) par un arrondi partiel (15).

# Fig. 1

# Fig. 2

# Fig.3

# Fig. 4